(19) Europäisches Patentamt
European Patent Office
Office européen des brevets

(11) Veröffentlichungsnummer: **0 069 210**
B1

(12) # EUROPÄISCHE PATENTSCHRIFT

(45) Veröffentlichungstag der Patentschrift: **12.09.84**

(51) Int. Cl.³: **C 07 C 47/21,** C 07 C 45/42

(21) Anmeldenummer: **82103712.4**

(22) Anmeldetag: **30.04.82**

(54) **Verfahren zur Herstellung von alpha, beta-ungesättigten Aldehyden und 2,7-Dimethyl-2,6-octadienal.**

(30) Priorität: **11.05.81 DE 3118656**

(43) Veröffentlichungstag der Anmeldung:
**12.01.83 Patentblatt 83/2**

(45) Bekanntmachung des Hinweises auf die Patenterteilung:
**12.09.84 Patentblatt 84/37**

(84) Benannte Vertragsstaaten:
**CH DE FR GB IT LI**

(56) Entgegenhaltungen:
**DE - A - 2 439 140**
**DE - A - 2 632 220**
**DE - A - 2 815 539**
**DE - A - 2 847 069**

(73) Patentinhaber: **BASF Aktiengesellschaft,**
***Carl-Bosch-Strasse* 38, D-6700 Ludwigshafen (DE)**

(72) Erfinder: **Fischer, Rolf, Dr., Bergstrasse 98,**
**D-6900 Heidelberg (DE)**
*Erfinder:* **Weitz, Hans-Martin, Dr., Auf dem Koeppel 40,**
**D-6702 Bad Duerkheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 069 210 B1

**Beschreibung**

Die Erfindung betrifft ein Verfahren zur Herstellung von α,β-ungesättigten Aldehyden durch Umsetzung von in 2-Stellung durch aliphatische Reste substituierten 1.4-Diacyloxy-2-alkenen mit Wasser in Gegenwart von Säuren sowie von 2,7-Dimethyl-2,6-octadienal.

Es ist aus Bull. Soc. Chim. France *11*, 218-223 (1944) bekannt, dass man bei der Umsetzung ungesättigter Diole mit verdünnter Schwefelsäure oder äquimolaren Mengen Phosphorsäure und Wasser Aldehydgemische erhält. So entstehen aus 2-Penten-1.4-diol Gemische von 2- und 3-Pentenal, aus 2-Hexen-1.4-diol Gemische von 2- und 3-Pentenal, aus 2-Hexen-1.4-diol Gemische von 2- und 3-Hexenal und aus 2.3-Dimethyl-2-buten-1.4-diol Gemische von 2.3-Dimethyl-2-butenal und 2.3-Dimethyl-3-butenal.

Aus der DE-AS 2815539 und der DE-AS 2847069 ist bekannt, dass durch Umsetzung von 2-Alkyl-2-buten-1.4-diol-diacylaten mit Wasser in Gegenwart von Kationenaustauschern oder Mineralsäuren 2-Alkylcrotonaldehyde entstehen.

Es wurde nun gefunden, dass man α,β-ungesättigte Aldehyde der Formel

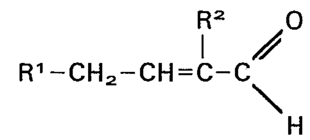

(I)

in der $R^1$ und $R^2$ aliphatische Kohlenwasserstoffreste mit 1 bis 15 Kohlenstoffatomen bedeuten, vorteilhaft erhält, wenn man 1.4-Diacyloxy-2-alkene der Formel

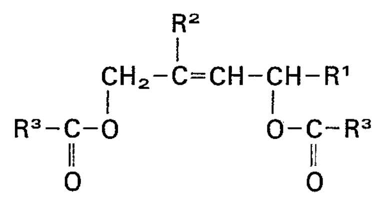

(II)

in der $R^1$ und $R^2$ die vorgenannte Bedeutung haben und $R^3$ ein Wasserstoffatom oder einen Alkyl-, Cycloalkyl- oder Arylrest bezeichnet, mit Wasser in Gegenwart von Mineralsäuren oder Kationenaustauschern umsetzt.

Die Umsetzung kann im Falle der Herstellung von 2.7-Dimethyl-2.6-octadienal aus 2,7-Dimethyl-1.4-diacetoxy-2.7-octadien durch die folgenden Formeln wiedergegeben werden:

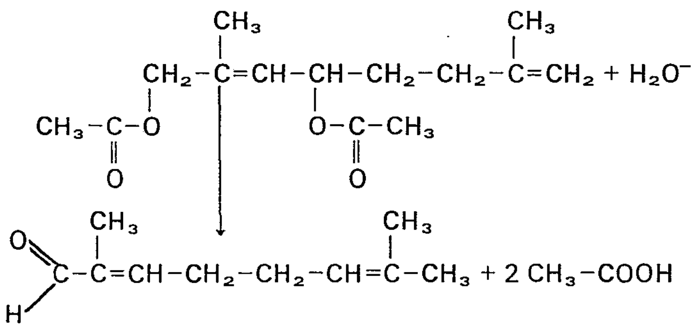

Das Verfahren nach der Erfindung liefert auf einfachem und wirtschaftlichem Wege α,β-ungesättigte Aldehyde. Die Aufarbeitung ist einfach, teure oder schwer zugängliche Reaktionskomponenten sind nicht nötig. Vorteilhaft kann das Verfahren in industriellem Massstab durchgeführt werden. Der Ausgangsstoff kann in einstufiger Reaktion ohne vorangehende Umsetzung zum Diol zu α,β-ungesättigten Aldehyden umgesetzt werden. Grosse Mengen an Salz infolge Neutralisation werden vermieden.

Es war aufgrund des folgenden technischen Sachverhaltes nicht vorauszusehen, wie die in 2- und 4-Stellung durch aliphatische Reste substituierten 1.4-Diacyloxy-2-butene der Formel II und/oder die darin durch Verseifung entstehenden Diole in Gegenwart von Wasser und Mineralsäuren oder Kationenaustauschern reagieren würden.

Nach Houben-Weyl, Methoden der Organischen Chemie, Band 6/3, Seiten 592 bis 594, bilden sich aus 1,4-Dihydroxy-2-buten in Gegenwart sauer reagierender Katalysatoren Dihydrofurane. Aus der DE-AS 2062950 ist bekannt, dass cis-2-Buten-1,4-diolmonoacetat oder -diacetat in Gegenwart von Säuren in 2,5-Dihydrofuran übergehen. Anderseits liefert cis-1.4-Dihydroxy-2-buten bei Behandlung mit wässeriger Schwefelsäure Crotonaldehyd (65%) und 2.5-Dihydrofuran (35%), während trans-1.4-Dihydroxy-2-buten mit quantitativer Ausbeute in Crotonaldehyd übergeht (M.A. Valette, Comptes Rendus *223*, 907 (1946)).

Während die Umsetzung von 2-Methyl-1.4-diacetoxy-2-buten mit Wasser in Gegenwart von Kationenaustauschern (DE-AS 2815539) oder Mineralsäuren (DE-AS 2847069) mit Ausbeuten von bis zu 98% zu 2-Methyl-2-butenal (Tiglinaldehyd) führt, werden bei der Umsetzung von 1.4-Diacetoxy-2-penten mit Wasser in Gegenwart von Kationenaustauschern nur Ausbeuten an 2-Pentenal von 29% erhalten (siehe Vergleichsbeispiel 1). 1.4-Dihydroxy-2-penten ergibt bei der Umsetzung mit 1 bis 25%iger Schwefelsäure oder äquimolaren Mengen Phosphorsäure und Wasser Gemische aus 2- und 3-Pentenal. Bei der entsprechenden Umsetzung von 1.4-Dihydroxy-2-hexen werden Gemische aus 2- und 3-Hexenal gebildet (Ch. Prévost, Bull. Soc. Chim. France *11*, 218 (1944)). 2.5-Dihydroxy-3-hexen lässt sich dagegen mit Chlorwasserstoff zu 2.5-Dimethyl 2.5-dihydrofuran umsetzen (Houben-Weyl, obengenanntes Zitat). 2.3-Dimethyl-1.4-diacetoxy-2-buten lässt sich im Gegensatz zu 2-Methyl-1.4-diacetoxy-2-buten mit Wasser in Gegenwart von Kationenaustauschern nur mit Ausbeuten von rund 17% in 2.3-Dimethyl-2-butenal überführen (siehe Vergleichsbeispiel 2). Cis- und trans-2.3 dimethyl-1.4-dihydroxy-2-buten ergeben dagegen mit Mineralsäuren Gemische aus 2.3-Dimethyl-2- und 2.3-Dimethyl-3-butenal (Ch. Prévost, obengenanntes Zitat).

In den Ausgangsstoffen der Formel II stehen die Reste $R^1$ und $R^2$ für aliphatische Kohlenwasserstoffreste, wie lineare oder verzweigte Alkyl-, Alkenyl- oder Alkadienylreste mit 1 bis 15, vorzugsweise 1 bis 10 Kohlenstoffatomen.

Solche Reste sind z.B. die Reste der Formeln $CH_3$, $C_2H_5$, n-$C_4H_9$, sek.-Butyl-,
$-CH_2-C(CH_3)=CH_2$, $-CH_2-CH=CH_2$,
$-(CH_2)_2-C(CH_3)=CH_2$,
$-CH_2-CH_2-CH=CH_2$, oder
$-CH_2-CH_2-CH=C(CH_3)_2$.

$R^3$ steht für ein Wasserstoffatom, für einen Alkyl- oder Cycloalkylrest mit 1 bis 10 Kohlenstoffatomen, wie einen Methyl- Ethyl-, Propyl-, n-Butyl-, i-Butyl-, n-Pentyl, i-Pentyl, n-Decyl-, Cyclopentyl-, Cyclohexylrest oder für einen Arylrest, wie einen Phenylrest. Geeignete Ausgangsstoffe der Formel II sind z.B. 2-Methyl-1.4-diacetoxy-2-penten, 2.7-Dimethyl-1.4-diacetoxy-2.7-octadien, 2,6-Dimethyl-1.4-diacetoxy-2.7-octadien, 2-Ethyl-1.4-diacetoxy-2-penten, 2-Methyl-1.4-diacetoxy-2-decen oder 2-(4-Methyl-3-pentenyl)-1.4-diacetoxy-2-penten.

Die Ausgangsstoffe der Formel II lassen sich aus den entsprechend substituierten 1.3-Dienen entweder durch Bromierung und nachfolgende Umsetzung der 1.4-Dibromverbindungen mit den Alkalisalzen der entsprechenden Carbonsäuren der Formel $R^3-COOH$ oder in einstufiger Reaktion durch Acyloxilierung der 1.3-Diene mit Carbonsäuren und mit Sauerstoff in Gegenwart von Katalysatoren, die z.B. Palladium und Tellur DE-PS 2217452), Platin und Tellur (DE-AS 2417558) oder Palladium und Kupfer (DE-AS 2820519) enthalten, herstellen.

Die Umsetzung wird im allgemeinen bei Temperaturen von 0 bis 200° C, vorzugsweise von 80 bis 120° C, drucklos oder unter Druck, diskontinuierlich oder kontinuierlich in Gegenwart von Wasser durchgeführt. Die Wassermenge beträgt vorteilhaft 1 bis 50, insbesondere 2 bis 20 Mol je Mol Ausgangsstoff der Formel II. Zusätzlich können unter den Reaktionsbedingungen inerte, organische Lösungsmittel verwendet werden. Hierfür kommen vor allem solche in Frage, die mit Wasser mischbar sind oder ein gutes Lösungsvermögen für Wasser aufweisen, z.B. Alkanole und Cycloalkanole wie Ethanol, Methanol, n-Butanol, Isobutanol, tert.-Butanol, Glykol, Glycerin, sek.-Butanol, n-Propanol, Isopropanol. Es hat sich auch als vorteilhaft erwiesen, in Gegenwart sogenannter Phasentransferkatalysatoren wie z.B. Tetraalkylammoniumhalogeniden zu arbeiten.

Die Umsetzung wird in Gegenwart von sauren Ionenaustauschern, vorzugsweise sauren Kunstharzaustauschern, die zweckmässig in ihrer Säureform vorliegen, durchgeführt. Solche Austauscher sind z.B. alle in Houben-Weyl, Methoden der Organischen Chemie, Band I/1, Seite 528, Tabelle 3, beschriebenen Kationenaustauscher. Vorzugsweise verwendet man stark und mittelstark saure Austauscher, z.B. Austauscher, die aus Styrol und Divinylbenzol aufgebaut sind und Sulfonsäuregruppen enthalten oder stark saure anorganische Kationenaustauscher wie Zeolithe, Phenol- oder Polystyrolsuflonsäureharze, Styrolphosphonsäureharze, Styrolphosphinsäureharze sowie entsprechende saure Harze enthaltende Austauscher, z.B. bifunktionelle Kondensationsharze. Als saure Harze der genannten Art kommen z.B. die im Handel unter der Bezeichnung "Lewatit S 100, "Amberlit IR-120, "Lewasorb, "Dowex 50 WX 8 und "Amberlyst 15 erhältlichen Produkte in Betracht. Die Menge der verwendeten Ausgangsstoffe bzw. des Austauschers hängt von der Selektivität bzw. der Zahl der austauschaktiven Gruppen der verwendeten Austauscher bei der Reaktionstemperatur ab. Im allgemeinen verwendet man 1 bis 40 Gewichtsprozent, vorzugsweise 5 bis 25 Gewichtsprozent Austauscher, bezogen auf den Ausgangsstoff der Formel II. Die im trockenen Austauscher enthaltene Wassermenge, die je nach Struktur bis zu 50 Gewichtsprozent betragen kann, ist in dem oben definierten Zusatz an Wasser nicht einbegriffen. Form und Korngrösse des Austauschers können in einem weiten Bereich beliebig gewählt werden. Bezüglich Herstellung und Einzelheiten der Verwendung von Ionenaustauschern wird auf das Kapitel «Ionenaustauscher» des genannten Werkes verwiesen.

Anstelle von Ionenaustauschern können auch Mineralsäuren wie Salzsäure, Schwefelsäure, Salpetersäure oder Bromwasserstoffsäure verwendet werden. Man verwendet 0,002 bis 0,25 Äquivalente der genannten Säure je Mol der Ausgangsverbindung der Formel II.

Die Umsetzung kann z.B. wie folgt durchgeführt werden: Ein Gemisch aus dem Ausgangsstoff der Formel II, Wasser, Mineralsäure oder dem sauren Kationenaustauscher, das gegebenenfalls ein organisches Lösungsmittel enthält, wird 0,5 bis 24 Stunden bei der Reaktionstemperatur gehalten. Dann wird der Endstoff der Formel I in üblicher Weise, z.B. nach Abfiltrieren des Kationenaustauschers und fraktionierter Destillation des Filtrats, abgetrennt. Die zugesetzten Mineralsäuren und geringen Mengen an Carbonsäuren, z.B. Essigsäure, können durch Neutralisation entfernt werden.

Besonders einfach können die Aldehyde der Formel I durch Abdestillieren und Kondensieren von Aldehyd-Wassergemischen in einer Vorrichtung zur kontinuierlichen Abnahme der oberen Destillatphase eines zweiphasigen Gemisches abgetrennt werden. Hierzu wird ein Gemisch aus der Ausgangsverbindung der Formel II, Wasser und der jeweiligen Säure oder dem Ionenaustauscher in einem Reaktionskolben auf die Reaktionstemperatur erwärmt. Das aus dem Reaktionsgemisch abdestillierende Gemisch aus Aldehyd und Wasser

wird kondensiert, wobei es sich in eine Aldehyd- und eine Wasserphase auftrennt. Das Wasser fliesst in den Reaktionskolben zurück, so dass die Wassermenge dort, abgesehen von dem bei der Aldehyd-Bildung verbrauchten Wasser, konstant bleibt. Das verbrauchte Wasser kann gegebenenfalls während er Umsetzung ergänzt werden. Im Rohprodukt noch enthaltene Mineral- und/oder Carbonsäure wird gegebenenfalls durch Neutralisation entfernt. Der Aldehyd wird gegebenenfalls nach Trocknung mit einem Trockenmittel fraktioniert destilliert.

Die nach dem Verfahren der Erfindung erhältlichen α,β-ungesättigten Aldehyde sind wertvolle Ausgangsstoffe für die Herstellung von Farbstoffen, Pflanzenschutzmitteln und pharmazeutischen Produkten. Sie können weiterhin als Aromabestandteile von Lebensmitteln und Getränken dienen (DT-AS 2927090 und 2937287). 2.7-Dimethyl-2.6-octadienal, das mit Citral isomer ist, kann wie dieses bei der Herstellung von Parfüms und Kosmetikartikeln Verwendung finden. Bezüglich der Verwendung von α,β-ungesättigten Aldehyden wird weiterhin auf Ullmanns Encyclopädie der technischen Chemie, 4. neubearbeitete und erweiterte Auflage, Band 7, S. 118, verwiesen.

Die in den folgenden Beispielen angegebenen Teile sind Gewichtsteile.

*Beispiele 1*

Es wird ein unter der Bezeichnung ® Amberlite IR-120 (20 bis 50 mesh, Wassergehalt 44 bis 48 Prozent) im Handel erhältlicher Polystyrolsulfonsäureharz-Kationenaustauscher verwendet.

Ein Gemisch aus 22,1 Teilen 2-Methyl-1,4-diacetoxy-2-penten, 80Teilen Wasser und 10 Teilen des wasserhaltigen Kationenaustauschers wird 2 Stunden lang auf 95 bis 100°C erhitzt, wobei gleichzeitig 2-Methyl-2-pentenal durch Abdestillieren und Kondensieren eines Aldehyd-Wassergemisches in einer Vorrichtung zur kontinuierlichen Abnahme der oberen Destillatphase eines zweiphasigen Gemisches abgetrennt wird. Die organische Phase wird nach Trocknen mit Magnesiumsulfat fraktioniert destilliert. Man erhält 6,8 Teile 2-Methyl-2-pentenal (63% der Theorie) vom Siedepunkt 133 bis 135°C /1010 mbar, $n_D^{20}$=1,4502.

*Beispiel 2*

Ein Gemisch aus 11 Teilen 2,7-Dimethyl-1.4-diacetoxy-2.7-octadien, 35 Teilen Wasser und 4,3 Volumenteilen 1N-Salzsäure (0,1 Äquivalente HCl, bezogen auf 1 Mol des Diacetats) wird 4 Stunden unter Rückfluss gekocht. Dann wird, wie in Beispiel 1 beschrieben, ein Aldehyd-Wassergemisch ausgekreist. Aus der so gewonnenen organischen Phase erhält man durch fraktionierte Destillation 4,3 Teile 2.7-Dimethyl-2.6-octadienal (65% d. Th) vom Siedepunkt 80 bis 82°C / 16 mbar, $n_D^{20}$=1,4776.

*Beispiel 3*

Man verfährt wie in Beispiel 2 beschrieben und erhält aus 4 Teilen 2.6-Dimethyl-1.4-diacetoxy-2.7-octadien, 15 Teilen Wasser und 1,6 Volumenteilen 1N-Salzsäure 1,4 Teile 2.6-Dimethyl-2.7-octadienal (58% d.Th.) vom Siedepunkt 60 bis 78°C /16 mbar, $n_D^{20}$=1,4710.

*Vergleichsbeispiel 1*

Es wird ein unter der Bezeichnung ®Dowex 50 WX 8 (50 bis 100 mesh) im Handel erhältlicher Polystyrolsulfonsäureharz-Kationenaustauscher verwendet. Er wird mit Salzsäure vorbehandelt und mit Wasser neutral gewaschen.

Ein Gemisch aus 20 Teilen 1-Methyl-1.4-diacetoxy-2-buten, 20 Teilen Wasser und 5 Teilen des Kationenaustauschers wird zwei Stunden unter Rückfluss erhitzt. Nach dem Abkühlen auf Raumtemperatur filtriert man den Ionenaustauscher ab. Die organische Phase des zweiphasigen Filtrats wird im Scheidetrichter abgetrennt und mit Natriumsulfat getrocknet. Bei der fraktionierten Destillation der 3,5 Teile organischer Phase werden 2,6 Teile 2-Pentenal (28,9% d. Th.) vom Siedepunkt 124 bis 126°C /1010 mbar, $n_D^{20}$=1,4359 erhalten.

*Vergleichsbeispiel 2*

Ein Gemisch aus 100 Teilen 2.3-Dimethyl-1.4-diacetoxy-2-buten, 100 Teilen Wasser und 27 Teilen Dowex 50 WX 8 wird 2 Stunden unter Rückfluss gerührt. Dann wird wie in Beispiel 1 beschrieben ein Gemisch aus Aldehyd und Wasser ausgekreist. Die so erhaltene organische Phase (30,4 Teile) liefert bei der fraktionierten Destillation 8,5 Teile rohes 2.3-Dimethyl-2-butenal (Rohausbeute 17% d.Th.) vom Siedepunkt 127 bis 170°C /1013 mbar.

**Patentanspruch**

Verfahren zur Herstellung von α,β-ungesättigten Aldehyden der Formel

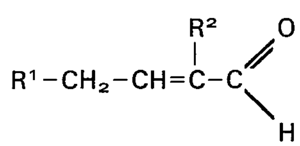

(I)

in der $R^1$ und $R^2$ aliphatische Kohlenwasserstoffrest mit 1 bis 15 Kohlenstoffatomen bedeuten, dadurch gekennzeichnet, dass man 1.4-Diacyloxy-2-alkene der Formel

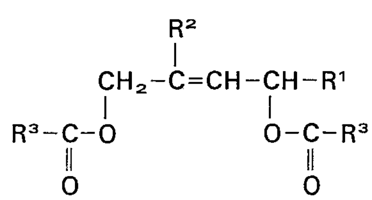

(II)

in der $R^1$ und $R^2$ die vorgenannte Bedeutung haben und $R^3$ ein Wasserstoffatom oder einen Alkyl-, Cycloalkyl- oder Arylrest bezeichnet, mit Wasser in Gegenwart von Mineralsäuren oder Kationenaustauschern umsetzt.

## Claim

A process for the preparation of an α,β-unsaturated aldehyde of the formula

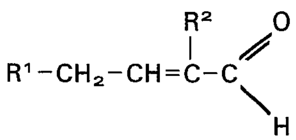

(I)

where $R^1$ and $R^2$ are aliphatic hydrocarbon radicals of 1 to 15 carbon atoms, wherein a 1.4-diacycloxyalk-2-ene of the formula

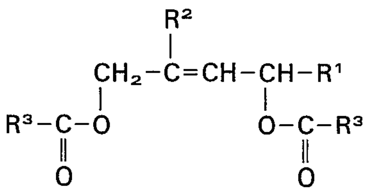

(II)

where $R^1$ and $R^2$ have the above meanings and $R^3$ is hydrogen, alkyl, cycloalkyl or aryl, is reacted with water in the presence of a mineral acid or cation exchanger.

## Revendication

Procédé de préparation d'aldéhydes alpha, bêta-insaturés de formule

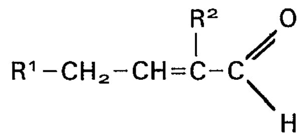

(I)

dans laquelle $R^1$ et $R^2$ représentent des restes hydrocarbonés aliphatiques en $C_1$-$C_{15}$, caractérisé en ce que l'on fait réagir des 1.4-diacyloxy-2-aclcènes de formule

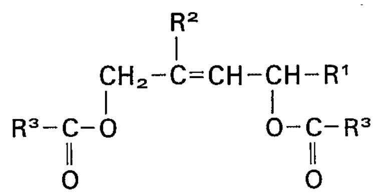

(II)

dans laquelle $R^1$ et $R^2$ ont les significations indiquées ci-dessus et $R^3$ représente un atome d'hydrogène ou un groupe alkyle, cycloalkyle ou aryle, avec l'eau en présence d'acides minéraux ou d'échangeurs de cations.